# EUROPEAN PATENT APPLICATION

(11) **EP 1 722 228 A1**
(43) Date of publication of application: **15.11.2006**
(21) Application number: 05709934.3
(22) Date of filing: 09.02.2005
(51) Int. Cl.: G01N 33/547

(54) **IMMOBILIZED BIOMOLECULE AND METHOD OF DETECTING SUBSTANCE CAPABLE OF INTERACTING WITH BIOMOLECULE**

(30) Priority: 05.03.2004 JP 2004061798; 02.11.2004 JP 2004319087
(71) Applicant: Nisshinbo Industries, Inc., Chuo-ku, Tokyo 103-8650 (JP)
(72) Inventor: AKIYAMA, Megumi, c/o Nisshinbo Industries, Inc., Chiba-shi, Chiba 2670056 (JP); KIMURA, Naoki, c/o Nisshinbo Industries, Inc., Chiba-shi, Chiba 2670056 (JP)
(74) Representative: Tombling, Adrian George
(86) International application number: PCT/JP2005/001882
(87) International publication number: WO 2005/085857

(57) **Abstract**

It is an object of the present invention to provide a method of conveniently efficiently and easily immobilizing a protein on a substrate, a method of detecting a substance capable of interacting with the protein at a high sensitivity, and a protein and a protein-immobilized substrate which are used in these methods.

In the method of detecting a substance capable of interacting with a protein using the immobilized protein of the present invention, a protein bound to a compound having a group capable of binding onto a substrate for immobilizing a biomolecule or a carrier provided on the substrate and immobilized onto the substrate, is used as the immobilized protein.

## Description

### Technical Field

The present invention relates to detection of a substance capable of interacting with an immobilized biomolecule using the immobilized biomolecule. More specifically, the present invention relates to a method of detecting a substance capable of interacting with an immobilized biomolecule using the immobilized biomolecule, and to a biomolecule and biomolecule-immobilized substrate which are used for the method.

### Background Art

Conventionally, techniques for immobilizing a nucleic acid or protein on a support such as a membrane or plate are used in analyses of nucleic acids by hybridization, immunoassays, or the like, and among those techniques, the following are known as methods of immobilizing proteins (Non-Patent Document 1).

(1) A method of physically adsorbing a protein on a nitrocellulose membrane or poly-L-lysine.
(2) A method of immobilizing a protein by preparing a base material by introducing an aldehyde or epoxy group to a surface of a base material made of glass or the like and then reacting such functional groups with an amino group of the protein.
(3) A method of immobilizing a protein on a gold base material using difunctional thiolalkylene.

However, the method (1) has disadvantages, that is, the protein is easy to peel off from the substrate because the immobilization reaction is performed using physical adsorption, and higher background noises may be observed due to unspecific adsorption.

Meanwhile, the method (2) may overcome a disadvantage in that the protein peels off from the surface of the substrate because of covalent bond formation, but it requires a harmful reagent such as a reductant for the immobilization reaction and also requires skilled operations to obtain reproducible data.

In addition, the method (3) has difficulty in obtaining reproducible and quantitative data because the gold-thiol bond is formed by physical adsorption and the thiol group itself has poor stability.

Meanwhile, as a method of immobilizing a nucleic acid, there is known a technique of binding a nucleotide polymer or the like to a nucleic acid and binding it to a substrate for immobilization by irradiation with an ultraviolet ray or the like (Patent Document 1).
Non-Patent Document 1: Zhu, H. and Snyder, M. (2003) Protein chip technology. Curr. Opin. Chem. Biol. 7, 55-63.
Patent Document 1: JP 2001-281246 A.

### Disclosure of the Invention

### Problems to be solved by the Invention

It is an object of the present invention to provide a method of conveniently efficiently and easily immobilizing a biomolecule on a substrate, a method of detecting a substance capable of interacting with the biomolecule at a high sensitivity, and a biomolecule and a biomolecule-immobilized substrate which are used in these methods.

### Means for solving the Problems

To solve the above-mentioned problems, the inventors of the present invention have made studies, and as a result they have found out that: a biomolecule bound to a polymer containing a compound having an unsaturated bond or a compound having a photoreactive group may be firmly immobilized; and use of the thus-obtained biomolecule-immobilized substrate enables improvement of detection sensitivity of a substance capable of interacting with the biomolecule, thereby completing the present invention.

That is, the present invention is as follows:
(1) A biomolecule (excluding a nucleic acid) to be immobilized and used for a method of detecting a substance capable of interacting with the biomolecule using the immobilized biomolecule, wherein the biomolecule is bound to a compound having a group capable of binding onto a substrate for immobilizing a biomolecule to which the biomolecule is immobilized or a carrier provided on the substrate.
(2) A biomolecule according to (1), wherein the compound having a group capable of binding onto a substrate for immobilizing a biomolecule or a carrier provided on the substrate is a polymer that includes a compound having an unsaturated bond.
(3) A biomolecule according to (2), wherein the polymer has an average degree of polymerization of 2 or more to 1, 000, 000 or less.
(4) A biomolecule according to (2) or (3), wherein a monomer constituting the polymer is a nucleotide.
(5) A biomolecule according to (1), wherein the compound having a group capable of binding onto a substrate for immobilizing a biomolecule or a carrier provided on the substrate is a compound having at least one photoreactive group, and selected from compounds each having a nitrene precursor, carbene precursor, or ketone group.
(6) A biomolecule according to any one of (1) to (5), wherein the biomolecule is selected from a protein, sugar, antigen, antibody, peptide, and enzyme.
(7) A substrate for immobilizing a biomolecule, comprising: a substrate for immobilizing a biomolecule; and a biomolecule according to any one of (1) to (6) immobilized on the substrate.
(8) A method of producing a substrate for immobilizing a biomolecule, comprising: contacting the biomolecule according to any one of (1) to (6) with a substrate for immobilizing a biomolecule; and irradiating a contact portion with an electromagnetic ray.
(9) A method of detecting a substance capable of interacting with an immobilized biomolecule using the immobilized biomolecule, wherein the substrate for immobilizing a biomolecule according to (7) is used.

### Effect of the Invention

The present invention provides a biomolecule that may be stably immobilized on a substrate or a carrier provided on the substrate. Addition of a compound having a group capable of binding on a substrate or a carrier provided on the substrate to an arbitrary biomolecule enables increasing an amount of an arbitrary biomolecule that may be immobilized on a substrate or a carrier provided on the substrate, resulting in improvement of detection sensitivity.

### Best Mode for carrying out the Invention

Hereinafter, embodiments of the present invention will be described in detail.
<1> Biomolecule
The biomolecule of the present invention is a biomolecule bound to a compound having a group capable of binding onto a substrate for immobilizing a biomolecule or a carrier provided on the substrate. Examples of the biomolecule include protein, sugar, antigen, antibody, peptide, and enzyme.

Hereinafter, a description will be made of a protein as an example of the biomolecule, but even in the case of other substances, the present invention can be performed by using methods or conditions generally used for immobilization except that a compound having a group capable of binding onto a substrate for immobilizing a biomolecule or a carrier provided on the substrate is bound.

The protein of the present invention is almost the same as an immobilized protein to be used for a general method of detecting a substance capable of interacting with an immobilized protein using the immobilized (solid-phased) protein (such as immunoassay) except that the protein is bound to a compound having a group capable of binding onto a substrate for immobilizing a biomolecule or a carrier provided on the substrate, the protein is not particularly limited as long as it is a protein that may bind to a substance capable of interacting with a protein, and examples thereof include natural or synthetic proteins. Meanwhile, the size of the protein is not particularly limited as long as it allows binding with a substance capable of interacting with a protein.

In the protein, the portion to be bound to a compound having a group capable of binding onto a substrate for immobilizing a biomolecule or a carrier provided on the substrate is a portion of an amino or carboxyl terminal, side-chain amino group, side-chain carboxyl group, side-chain thiol group, side-chain alcohol group, or the like in the protein.

As a method of binding a compound having a group capable of binding onto a substrate for immobilizing a biomolecule or a carrier provided on the substrate to a protein, a known method may be used. Examples of the method include: a method of binding the compound to a protein using a commercially available crosslinking agent; and a method of binding the compound to a protein by reacting a functional group of the protein with an atom, functional group, or structure reactive with the functional group.

Examples of the method of binding a compound having a group capable of binding onto a substrate for immobilizing a biomolecule or a carrier provided on the substrate to a protein using a crosslinking agent include the following method. An intended peptide is synthesized using a commercially available peptide synthesizer. As a compound that forms a polymer, a nucleotide is synthesized by polymerizing at least two bases of one or two or more kinds of nucleotides selected from ones having nucleic acid bases such as adenine, cytosine, thymine, and uracil using a commercially available nucleic acid synthesizer, and an amino group is introduced thereto using a commercially available reagent for introducing an amino group. The above-mentioned peptide and amino group-introduced nucleotide are bound using a commercially available crosslinking agent (such as DSS (Disuccinimidyl suberate)) in accordance with a conventional method.

Meanwhile, examples of a method of binding a compound to a sugar, the compound having a group capable of binding onto a substrate for immobilizing a biomolecule or a carrier provided on the substrate, include the following method. A hydroxyl group at position 6 of glucose is protected with a trityl group, and the other hydroxyl groups are acetylated, and then an azide group is introduced to position 6, followed by conversion into an amino group. Subsequently, an excessive amount of DSS is added to the aminated glucose, and purification is performed. Thereafter, a nucleotide prepared as above, to which a 5' -end or 3' -end amino group has been introduced, such as polydeoxyadenylic acid, polydeoxycytidylic acid, polydeoxythymidylic acid, or polydeoxyuridylic acid or a complex polymer thereof is allowed to react with a succinimidyl group of the above-mentioned aminated glucose. Deacetylation is performed using sodium methoxide, to thereby synthesize glucose to which a polymer has been introduced.

Specific examples of the atom, functional group, or structure reactive with a functional group of a protein include the following: as the atom, functional group, or structure reacted with an amino group, an isothiocyanate group; an imide ester group in a succinimide ester, a sulfonylsuccinimide ester, and the like; a halogen in a sulfonyl halide group or an alkyl halide group, and the like; an isocyanate group; an alkyl halide group in nitrogen yperite, and the like; a platinum complex; a carbodiimide group; an aldehyde group; an azide group; a cyano group in oxyimino-phenyl acetonitrile, and the like; a methylthio group in dimethylthiopyrimidine, and the like; and a diester group in dicarbonate and the like;
as the atom, functional group, or structure reacted with a carboxy group, a diazo group in diazoalkane, and the like; a halogen in an alkyl halide, and the like; a sulfonyl group in trifluoromethanesulfonate, and the like; a carbodiimide group; a hydrazino group in hydrazine, and the like; a phenacyl group in a phenacyl ester, and the like; a hydroxy group in 4-sulfo-2,3,5,6-tetrafluorophenol, and the like; and alkyltrifluoromethanesulfonate;
as the atom, functional group, or structure reacted with a thiol group, an iodine in iodoacetamide, and the like; an imide group in a mallein imide, and the like; a halogen in an alkyl halide, and the like; an aziridino group in aziridine, and the like; an epoxy group; a disulfide group in symmetric disulfide, and the like; a vinyl group in vinyl sulfone, and the like; and bromine in bromopyruvate and the like;
as the atom, functional group, or structure reacted with an alcohol group, 2-oxonitrile; an acid chloride; and an isocyanate group.

Examples of the method of binding the compound to a protein by reacting a functional group of the protein with an atom, functional group, or structure reactive with the functional group of the protein include the following method. A carboxyl group in the protein is activated with cesium carbonate, sodium, carbodiimide, thionyl chloride, or the like, and then a halogen or amino group in a compound having a group capable of binding onto a substrate for immobilizing a biomolecule or a carrier provided on the substrate is allowed to react in accordance with a conventional method.

Moreover, the protein of the present invention may be prepared by: previously immobilizing an amino acid bound to a compound having a group capable of binding onto a substrate for immobilizing a biomolecule or a carrier provided on the substrate, i.e., a compound having a polymer containing a compound having a unsaturated bond or a photoreactive group, on a substrate or a carrier provided on the substrate by irradiation with an electromagnetic wave or the like as described below; and then binding (elongating) any additional plural amino acids to the amino acids immobilized on the substrate or the carrier provided on the substrate by Spot synthesis method (Heine N, Germeroth L, Schneider-Mergener J, Wenschuh H: A modular approach to the spot synthesis of 1,2,5-trisubstituted hybridizations on cellulose membranes. Tetrahedron Lett 2001, 42:227-230), photolithography technique (Fodor SPA, Read JL, Pirrung LC, Stryer L, Lu AT, Solas D: Light-directed, spatially addressable parallel chemical synthesis. Science 1991, 251:767-773.), Fmoc method (Hasegawa K, Sha YL, Bang JK, Kawakami T, Akaji K, Aimoto S: Preparation of phosphopeptide thioesters by Fmoc- and Fmoc(2-F)-solid phase synthesis. Lett Pept Sci 2002, 8:277-284.), or the like.

In a compound having a group capable of binding onto a substrate for immobilizing a biomolecule or a carrier provided on the substrate, the group capable of binding onto a substrate or a carrier provided on the substrate is not particularly limited as long as it is reactive with a substrate or a carrier provided on the substrate and forms a firm bond such as a covalent bond. The group capable of binding onto a substrate or a carrier provided on the substrate may be contained in a compound in an amount sufficient to immobilize a protein on a substrate for immobilizing a protein, and one or more of the groups may be contained. Examples of the compound having a group capable of binding onto a substrate for immobilizing a biomolecule or a carrier provided on the substrate include a polymer that includes a compound having an unsaturated bond and a compound having a photoreactive group.

The polymer that includes a compound having an unsaturated bond means that at least one of monomers which composes a polymer is a compound having an unsaturated bond. The compound having an unsaturated bond may be contained in an amount sufficient to immobilize a polymer on a substrate for immobilizing a protein. Meanwhile, all monomers that are components of the polymer may be compounds having unsaturated bonds. Note that the phrase "including a compound having an unsaturated bond" means being composed of a residue of a compound having an unsaturated bond or including the residue.

The average degree of polymerization of the polymer, which represents the length thereof, is preferably 2 to 1,000,000, more preferably 5 to 100,000, particularly preferably 7 to 1,000.

If the average degree of polymerization is 1 or less, a sufficient amount of a protein may not be immobilized on a support, while if the average degree of polymerization is 1, 000, 001 or more, measurement may be prevented because a target molecule cannot approach a protein due to steric hindrance.

Specific examples of the polymer include a polymer containing a monomer selected from the group consisting of: a nucleotide having as bases adenine, an adenine derivative, cytosine, a cytosine derivative, guanine, a guanine derivative, thymine, a thymine derivative, uracil, and an uracil derivative; an ester-based monomer of acrylic acid or methacrylic acid; a styrene-based monomer; a polyolefin-based monomer; a vinyl-based monomer; a nitrile-based monomer; ethyleneglycol diacrylate; ethyleneglycol dimethacrylate; tetraethyleneglycol diacrylate; trimethylol propanetriacrylate; tetramethylol propanetetraacrylate; dipentaerythritol pentaacrylate, and the like. The kind of the monomer in the polymer may be identical to or different from each other. The preferable monomer is a nucleotide, and the particularly preferable monomer is a nucleotide having adenine as a base, a nucleotide having cytosine as a base, a nucleotide having thymine as a base, and a nucleotide having uracil as a base.

In a compound having a photoreactive group, the photoreactive group means a group that generates a group having high reactivity to a substrate or a carrier provided on the substrate by irradiation with light.

The compound having a photoreactive group may be one molecule or a polymer including a compound having a photoreactive group. The polymer including a compound having a photoreactive group means that at least one of monomers which composes of a polymer is a compound having a photoreactive group. The compound having a photoreactive group may be contained in an amount sufficient to immobilize a protein on a substrate for immobilizing a protein. Meanwhile, all monomers that are components of the polymer may be compounds having photoreactive groups.

Specific examples of the photoreactive group include a nitrene precursor, carbene precursor, and ketone group. Herein, the precursor means a group capable of generating active species such as nitrene and carbene. The compound having a photoreactive group includes any one or two or more photoreactive groups of various photoreactive groups. Specific examples of the compound having a photoreactive group include an allyl ketone, azide, or diazo compound.

To maintain an appropriate distance between a biomolecule to be immobilized and the surface of the substrate so that an immobilized biomolecule can sterically sufficiently approach a target molecule, a spacer compound may be introduced to a compound having a group capable of binding onto a substrate for immobilizing a biomolecule or a carrier provided on the substrate. As the spacer compound, there may be used a compound having a non-photoreactive atom, functional group, or structure capable of binding with a compound having a group capable of binding onto a substrate for immobilizing a biomolecule and a photoreactive biomolecule or a carrier provided on the substrate. The compound having a non-photoreactive atom, functional group, or structure is not particularly limited as long as it includes arbitrary at least two atoms, functional groups, or structures selected from, for example, alkyl group, cycloalkyl group, halogen, hydroxyl group, ether group (including haloether group), aldehyde group, carbonyl group, ester group, amide group, imide group, carboxyl group, sulfonyl group, phosphonyl group, nitro group, amino group, thiol group, and the like.

Note that the compound having a group capable of binding onto a substrate for immobilizing a biomolecule or a carrier provided on the substrate may include a polymer including the above-mentioned compound having a photoreactive group and a compound having an unsaturated bond.

To the biomolecule of the present invention may be introduced a compound having a group capable of binding onto a substrate for immobilizing a biomolecule or a carrier provided on the substrate as well as a photocrosslinking agent such as vinylated deoxyguanosine, vinylated deoxyguanosine derivative, psoralen, or psoralen derivative (such as 4' 5' -dihydropsoralen, 4, 5' 8-trimethylpsoralen, or angelicin), and such photocrosslinking agent may be used to three-dimensionally immobilize a biomolecule on a solid-phase support.

<2> Substrate for immobilizing protein
A substrate to be used as the substrate for immobilizing a protein of the present invention is not particularly limited as long as it is reactive with a compound having a group capable of binding onto a substrate for immobilizing a biomolecule or a carrier provided on the substrate, may immobilize a protein bound to such a compound by a chemical bond, and has resistance to conditions of a general method of detecting a substance capable of interacting with an immobilized-protein using the general immobilized-protein. Specific examples thereof include a substrate that is insoluble in a solvent to be used for immobilization of a protein, a method of detecting a substance capable of interacting with an immobilized-protein, and the like and is in the form of a solid or gel at ordinary temperature or at a temperature range near ordinary temperature (for example, 0 to 100°C). Note that the phrase "a substrate is insoluble in a solvent" means that a substrate is substantially insoluble in various solvents such as aqueous solvents and organic solvents, which is used in steps for: carrying a carrier having a group such as carbodiimide group that has binding ability to a protein on a substrate as described below; immobilizing a protein; and using the substrate as a protein tip or the like.

Specific examples of materials of such a substrate include plastics, inorganic polymers, metals, and ceramics.

Specific examples of the above-mentioned plastics include synthetic resins such as thermoplastic resins, thermosetting resins, and copolymers and natural resins.

Specific examples of the thermoplastic resin include: polycarbodiimide; ionomer such as a styrene-based ionomer, and an olefin-based ionomer; polynorbornene; polyacetal; polyarylate; polyether etherketone; polyethylene oxide; polyoxymethylene; polyethylene terephthalate; polycarbonate; polystyrene; polysulfone; polyparamethylstyrene; polyallylamine; polyphenylene ether; polyphenylene sulfide; polybutadiene; polybutylene terephthalate; polypropylene; polymethylpentene; polyether sulfone; polyphenylene sulfide; polyoxybenzoyl; polyoxyethylene; cellulose acetate; polydimethyl siloxane; polyisobutylene; cellulose triacetate; poly-p-phenylene terephthalamide; polyisoprene; polyacrylonitrile; polymethylpentene; a chlorinated plastic such as polyvinyl chloride, polyethylene chloride, chlorinated polypropylene, and polyvinylidene chloride; a fluorinated plastic such as tetrafluoroethylene, polychlorotrifluoroethylene, and polyfluorinated vinylidene; nitrocellulose; polyamide such as nylon 6 and nylon 66; polyamide imide; polyimide such as thermoplastic polyimide, and polyether imide; polyethylene plastic such as a chlorinated polyethylene plastic, a high density polyethylene plastic, and a low density polyethylene plastic; a polyvinyl plastic such as polyvinyl chloride, polyvinyl acetate, polyparavinyl phenol, polyvinyl alcohol, polyvinyl ether, polyvinyl butyral, and polyvinyl formal; a liquid crystal polymer such as a polyester-based liquid crystal polymer; an acrylate plastic such as aminopolyacrylamide, polyacrylamide, polymethyl methacrylate, ethyl polymethacrylate, and butyl polymethacrylate; and thermoplastic elastomer such as a styrene-based thermoplastic elastomer, an olefin-based thermoplastic elastomer, an urethan-based thermoplastic elastomer, a polyester-based thermoplastic elastomer, a polyamide-based thermoplastic elastomer, a 1,2-butadiene-based thermoplastic elastomer, a vinyl chloride-based thermoplastic elastomer, a fluorine-based thermoplastic elastomer, a polyionomer-based thermoplastic elastomer, a chlorinated polyethylene-based thermoplastic elastomer, and a silicone-based thermoplastic elastomer.

Specific examples of the thermosetting plastic include: an epoxy; polyxylene; polyguanamine; polydiallylphthalate; a polyvinyl ester; polyphenol; an unsaturated polyester; polyflan; polyimide; polyurethane; polymaleic acid; melamine; urea; alkyd; benzoguanamine; polycyanate; and polyisocyanate.

Further, a copolymer can be used as the plastic. Specific examples of the copolymer include: an isobutylenemaleic anhydride copolymer; an acrylonitrile acrylate styrene copolymer; an acrylonitrile EPDM styrene copolymer; an acrylonitrile styrene copolymer; an acrylonitrile butadiene styrene copolymer; a butadiene styrene methylmethacrylate copolymer; an ethylene vinyl chloride copolymer; an ethylene vinyl acetate copolymer; an ethylene-ethyl acrylate copolymer; an acrylonitrile-butadiene styrene copolymer; a polyetheretherketone copolymer; an ethylene floride polypropylene copolymer; a tetrafluoroethylene perfluoroalkyl vinyl ether copolymer; and a tetrafluoroethylene ethylene copolymer.

Moreover, more specific examples of the natural resins include cellulose, rosin, copal, dammar, Canada balsam, elemi, sandarac, gutta percha, sumac, shellac, amber, bast fiber, leaf fiber, fruit fiber, fur fiber, cocoon fiber, feather fiber, chitin, chitosan, asbestos, and derivatives thereof.

Meanwhile, there may be used a synthetic resin prepared by optionally adding a dye, colorant, plasticizer, pigment, polymerization inhibitor, surface-modification agent, stabilizer, adhesion-imparting agent, thermosetting agent, dispersant, ultraviolet degradation inhibitor, or the like to the above-mentioned synthetic resin. In addition, the above-mentioned synthetic resin may be formed by laminating different kinds of the above-mentioned synthetic resins to maintain its shape or may be made of a single synthetic resin. Moreover, it may be a polymer alloy formed by mixing two or more kinds of the above-mentioned synthetic resins.

Meanwhile, specific examples of the inorganic polymers include glass, quartz, carbon, silica gel, and graphite.

In addition, preferable examples of the metals include: metals selected from elements of the I, II, III, IV, V, VI, VII, VIII groups in the second to seventh periods of the periodic table, and transition elements; or alloys containing such metals.

Particularly preferable examples of the above-mentioned metals selected from elements of the I, II, III, IV, V, VI, VII, VIII groups in the second to seventh periods of the periodic system, and transition elements include aluminum, titanium, platinum, tungsten, molybdenum, gold, copper, and nickel.

In addition, specific examples of the alloy include: a white metal composed of Cu, Ni, and Zn; brass composed of Cu and Zn; bronze composed of Cu and Be; monel composed of Cu, Ni, Fe, and Mn; a nickel cobalt alloy composed of Ni and Co; a nickel chrome alloy composed of Ni and Cr; a cobalt alloy composed of Co, Ni, and Cr; stainless composed of Ni, Cr, and Fe; silver tungsten composed of Ag and W; b titanium composed of Ti, V, and Al; ab titanium composed of Ti, V, and Al; an NT alloy composed of Ti and Ni; an aluminium alloy composed of Al, Cu, Mg, Si, Mn, and Zn; duralumin composed of Al, Cu, Si, Fe, Mn, Mg, and Zn; a magnesium alloy composed of Mg, Al, and Zn; K24 composed of Au; K18 composed of Au, Ag, and Cu; beryllium copper composed of Cu and Be; casting iron composed of Fe, Mn, S, and C; carbon steel composed of Fe, C, Si, Mn, P, and S; a bronze cast composed of Cu, Sn, Zn, and Pb; a phosphor bronze cast composed of Cu, Zn, and P; a brass cast composed of Cu, Zn, and Pb; manganese brass composed of Cu, Zn, Mn, Fe, and Al; a silzin bronze cast composed of Cu, Si, and Zn; an aluminum bronze cast composed of Cu, Al, Fe, Ni, and Mn; Elinvar composed of Ni, Cr, and Mn; Elinvar extra composed of Ni, Cr, Co, and Mn; Inver composed of Ni and Fe; Super inver composed of Fe, Ni, and Co; stainless inver composed of Fe, Co, and Cr; Malottes composed of Sn, Bi, and Pb; Lipowitz composed of Sn, Bi, Pb, and Cd; Wood's composed of Sn, Bi, Pb, and Cd; Manganin composed of Cu, Mn, Ni, and Fe;, Isaberin composed of Cu, Mn, and Al; Constantan composed of Cu and Ni; Alcres composed of Fe, Cr, and Al; Kanthar composed of Cr, Fe, Al, and Co; Alumel composed ofNi andAl; a magnetic material including a ferromagnetic transition element such as Fe, Ni, and Co; Permalloy composed of Fe and Ni; Alperm composed of Fe and Al; ferrite which is a complex oxide having Fe₂O₃ as a main component; Sendust composed of Fe, Si, and Al; Super sendust composed of Fe, Si, Al, and Ni; Alnico composed of Fe, Al, Ni, and Co; hydrogen storage metal such as a lanthanum nickel alloy composed of La and Ni; a Co-Cr-based alloy; an SnO₂-based oxide; an Nb-Ti alloy; a damping alloy such as an alloy material which reduce or absorb vibration, or block out diffusion of vibration, an Al-Zn superplastic alloy, a silent alloy, and nitinol; a material for electrode; and a material for semiconductor such as silicon, germanium, and potassium arsenide.

Meanwhile, the above-mentioned metals may be subjected to a deposition or plating treatment (process) with another metal. Moreover, the above-mentioned metals maybe laminated with different kinds of the above-mentioned metals to maintain its shape or may be used as a single metal.

In the case of using the above-mentioned metals as the substrate of the present invention, the substrate may be made of only a metal or may be formed by laminating a metal on a nonmetallic material by adhesion, deposition, plating, or the like.

Meanwhile, specific examples of the ceramics include apatite, alumina, silica, silicon carbide, silicon nitride, and boron carbide.

The shape of the above-mentioned substrate is not particularly limited but examples thereof include a foil, plate, wafer, filter, or beads shape. Meanwhile, the substrate may have a shape like a microtiter plate. In addition, the substrate may be used as a sticker by applying or coating a material to be used for a sticker such as an adhesive to the back of a plate or the like to facilitate storage of the results to be obtained. Meanwhile, the size thereof is not particularly limited.

In the case where a protein is immobilized on the above-mentioned substrate, the protein may be directly immobilized on the substrate, or a carrier may be carried on the substrate to immobilize the protein on the substrate via the carrier. As the carrier, there may be used one having binding ability to the above-mentioned compound having a group capable of binding onto a substrate for immobilizing a biomolecule or a carrier provided on the substrate. Herein, the term "carried" refers to a state that the above-mentioned carrier is not substantially released from a substrate in various solvents such as aqueous solvents and organic solvents which are used in such cases of immobilizing a protein on a carrier and using the protein-immobilized substrate as a protein tip, or the like.

The above-mentioned carrier to be used in the present invention may be carried by using physically adhering ability alone or may be chemically carried via a covalent bond or the like as long as it is carried on the above-mentioned substrate. Meanwhile, if desired, the above-mentioned carrier may be carried on the entire surface or part of the substrate as needed.

Examples of the carrier include organic low-molecular substances, plastics, inorganic polymers, metals, and ceramics.

Specific examples of the above-mentioned organic low-molecular substances include polylysine, silane-coupling agent having a primary amino group such as 3-aminotriethoxyaminosilane or 3-aminotrimethoxyaminosilane, imide group-containing compound such as maleinimide or succinimide ester, carbodiimide group-containing compound, isocyanate group-containing compound, isothiocyanate group-containing compound, nitrogen yperite group-containing compound, aldehyde group-containing compound, amino group-containing compound, carboxyl group-containing compound, and halogen-containing compound.

Specific examples of the plastics that may be used include the synthetic resins such as thermoplastic reins, thermosetting resins, and copolymers and natural reins as described as materials of the substrate.

Meanwhile, specific examples of the inorganic polymers include glass, quarts, carbon, silica gel, and graphite.

In addition, preferable examples of the metals include: metals selected from elements of the I, II, III, IV, V, VI, VII, VIII groups in the second to seventh periods of the periodic table, and transition elements; or alloys containing such metals as described above as materials of the substrate.

Particularly preferable examples of the above-mentioned metals selected from elements of the I, II, III, IV, V, VI, VII, VIII groups in the second to seventh periods of the periodic table and transition elements, include aluminum, titanium, platinum, tungsten, molybdenum, gold, copper, and nickel.

Meanwhile, specific examples of the ceramics include apatite, alumina, silica, silicon carbide, silicon nitride, and boron carbide.

Such carriers have high adhering ability to the above-mentioned substrates and are carried on a substrate by such adhering ability. Note that, in the case where the above-mentioned carriers are carried on a substrate by physically adhering ability, they are typically in the form of films.

As a method of carrying the above-mentioned carrier in the form of a film on the above-mentioned substrate, there may be used known methods such as spray, immersion, brushing, stamp, vapor deposition, coating by a film coater.

For example, a method of introducing a carbodiimide group (resin) to the entire surface of a glass substrate is as follows. First, an amino-substituted organoalkoxysilane such as 3-aminopropyltriethoxysilane is dissolved in an appropriate solvent, a glass substrate is immersed in the resultant solution under temperature condition of about 70 to 80°C for about 2 to 3 hours, and then the substrate is taken out and washed with water, followed by heat drying at about 100 to 120°C for about 4 to 5 hours. After drying, the substrate is immersed in an appropriate solvent, a carbodiimide resin is added thereto, and the mixture is stirred under temperature condition of about 30 to 170°C for about 12 hours, followed by washing. Meanwhile, a nitrogen yperite group may be introduced to the surface of the glass substrate by reacting an amino group of the above-mentioned 3-aminopropyltriethoxysilane with a functional group other than protein-binding groups of the nitrogen yperite group using an appropriate solvent.

Meanwhile, conventionally, introduction of various functional groups to the surfaces of various materials exemplified in the above-mentioned description of substrates has been generally performed, and the methods thereof are also known, so even in the case where a functional group other than an amino group is introduced to a glass substrate, or even in the case where a substrate is made of a material other than glass, a functional group may be introduced to the surface of the substrate by such known methods.

Moreover, among the plastic substrates of the above-mentioned substrates, some of them already have the substrate surfaces with the functional groups as described above, and they may be used for producing supports without modification, i.e., without introduction of such functional groups to the substrate surfaces. Meanwhile, even in the case of such plastic substrates, they may be used for producing the above-mentioned supports after introducing additional functional groups.

Meanwhile, a photopolymerization initiator may be mixed in the above-mentioned carrier or substrate, or materials of the carrier and substrate. Mixing of a photopolymerization initiator may improve reactivity in immobilizing a protein by irradiation with an electromagnetic ray such as an ultraviolet ray.

<3> Protein-immobilized substrate
A protein solution is spotted on predetermined positions on the above-mentioned support. Examples of the protein include, but are not limited to, the proteins described in item <1> above.

A solvent to dissolve a protein is also not particularly limited, and examples of a solvent that may be used include distilled water or buffers to be generally used in preparation of a protein solution, for example, a Tris buffer, a PBS buffer (137 mM NaCl, 2.7 mM KCl, 1.5 mM KH₂PO₄, 8.1 mM Na₂HPO₄), a phosphonic acid-containing aqueous solution, a salt-containing aqueous solution, and a solvent that contains an anion selected from compounds containing a carboxyl, sulfonyl, or phosphonyl group and a cation selected from alkali metal, alkali earth metal, and onium ions. Use of such a solvent containing anion and cation enables efficient immobilization of a protein to a support by an electromagnetic ray. Specific examples of such a solvent containing anion and cation include carboxylate-containing aqueous solutions (such as sodium citrate, ammonium citrate, and sodium acetate), sulfonate-containing solutions (such as sodium dodecyl sulfate and ammonium dodecyl sulfate), and phosphonate-containing aqueous solutions (such as sodium phosphate and ammonium phosphate). Meanwhile, the concentration of the protein solution is also not particularly limited and is generally 1 mmol/µl to 1 fmol/µl, preferably 100 µmol/µl to 100 fmol/µl.

Examples of a method of spotting a protein solution on a support include a method of spotting by adding a protein solution dropwise on a support using a pipette or a method of spotting a protein solution by using a commercially available spotter. The spot shape and spot amount are not particularly limited as long as the positions which have been spotted with a protein solution are confirmed, and the shape is preferably dot-like or circle-like. Meanwhile, the spot amount is preferably 10 nl to 10 ml. The protein solution is spotted on one point or plural points on a support. One or two or more kinds ofproteinsolutionsmaybe spotted. Note that, as a positive control that proves immobilization of a protein on a support, a labeled protein may be immobilized.

After spotting a protein solution on a support, the support is irradiated with an electromagnetic ray, preferably an ultraviolet ray. Meanwhile, the above-mentioned protein solution may be dried after spotting before irradiation with an ultraviolet ray. Examples of a method of drying the above-mentioned protein solution include air drying and heat drying. In the case of heat drying, the temperature is generally 30 to 100°C, preferably 35 to 45°C.

In the case of immobilizing a protein using a compound having an unsaturated bond, an ultraviolet ray to be used for irradiation to a support, at least a protein-immobilized part on a support is preferably an ultraviolet ray containing a component having a wavelength of 240 to 380 nm. Specifically, it may be an ultraviolet ray that has a broad waveform including a wavelength of 254 or 335 nm. The irradiation amount is, as an accumulated irradiation amount, generally 20 to 2,400 mJ/cm², preferably 50 to 900 mJ/cm².

In the case of immobilizing a protein using a photocrosslinking agent and/or a compound having a photoreactive group, the wavelength, irradiation amount, or the like of the ultraviolet ray to be used for irradiation to a support, at least a protein-immobilized part on a support may be arbitrarily determined depending on the used photocrosslinking agent and/or compound having a photoreactive group.

In the case where the substrate for immobilizing a protein of the present invention is used for analysis or the like, a protein or the like other than the above-mentioned immobilized protein is often brought into contact with the substrate. However, to prevent nonspecifically binding of a protein or the like other than the above-mentioned immobilized protein to unreacted protein-immobilizing parts carried on a substrate or a carrier provided on the substrate, it is preferable that a protein is immobilized in dot-like form on a substrate or a carrier provided on the substrate as described above, and then the unreacted protein-immobilizing parts is blocked by contacting an excess amount of bovine serum albumin (BSA), casein, etc. with the substrate or the carrier provided on the substrate.

A substance to be detected by the detection method using a protein-immobilized substrate of the present invention is not particularly limited as long as it is a substance capable of interacting with an immobilized protein immobilized on the above-mentioned substrate for immobilizing a protein. Note that the term "interacting" refers to an intermolecular action between an immobilized protein and a substance, which is generated by a covalent bond, hydrophobic bond, hydrogen bond, van der Waals bond, bond caused by static electricity, or the like.

In the thus-obtained protein-immobilized substrate of the present invention, the above-mentioned protein is very firmly carried on a support and is not released by a washing method (such as a washing method using a surfactant) that is widely used in a method of detecting a substance capable of interacting with an immobilized protein using the immobilized protein (such as immunoassay) or the like, and in the case of analysis or the like using the protein-immobilized substrate, analysis with excellent reproducibility and quantitative ability may be performed. Meanwhile, in the protein-immobilized substrate of the present invention, a protein may be immobilized without limiting the size or kinds of the protein, so various proteins may be simultaneously treated on one substrate.

Based on such facts, the protein-immobilized substrate of the present invention is considered to be applicable to a protein tip (protein microarray) or the like with excellent performance.

### Example 1

### <Immobilization of peptide solution with solvent of aqueous solution of diammonium citrate on a support, and evaluation of peptide-immobilized support by antibody reaction>

In accordance with a conventional method, a peptide (7 residues) having an amino acid sequence shown in SEQ ID NO: 1 was synthesized using a peptide synthesizer. In addition, a peptide (7 residues) shown in SEQ I D NO: 2 in which tyrosine is phosphorylated was also synthesized. Note that the details of the peptide syntheses were referred to Fundament and Experiment of Peptide Synthesis (Peptide gousei no kiso to jikken) (Maruzen KK). Subsequently, an oligonucleotide obtained by introducing an amino group to the 5' -end of the oligonucleotide (10 residues) shown in SEQ ID NO: 3 was synthesized in accordance with an ordinary method.

The above-mentioned peptides were respectively dissolved with oligonucleotide in phosphate buffer (pH 7.5) at equimolar amounts, and 10-fold molar of DSS (manufactured by Pierce Biotechnology Inc.) was added, followed by incubation at 37°C for 2 hours. Subsequently, purification/concentration was performed using NAP5 column (manufactured by Amersham Biosciences), and the resultant was dissolved in an aqueous solution of 45 mM ammonium citrate, to thereby prepare a peptide solution (1 pmol/µl).

Each of the above-mentioned peptide solutions was spotted on predetermined positions on a support. The amount of each spotted solution was 0.5 µl, and the spot size was 1 mm in diameter. Subsequently, the support was irradiated with 600 mJ/cm² ultraviolet ray using Uvstratalinker 2400 (manufactured by Stratagene, center wavelength 254 nm) from a distance of 16 cm. The irradiation time was 240 seconds. Thereafter, the above-mentioned support was shaken in water for 30 minutes and washed, followed by drying.

On the other hand, a solution containing no peptide (aqueous solution of disodium citrate) was also spotted on a support as a control in the same way as above, and the operations for immobilization as described above were performed.

As a support, there was used a glass substrate treated with a polymer compound having a carbodiimide group.

On sample-immobilized parts on the above-mentioned support, a solution containing rhodamine-labeled anti-phosphorylated amino acid antibody (1 µg/100 µl antibody, 1 × PBS, 0.2% Tween 20, 1% BSA) was placed, and incubation was performed for 5 hours. The used rhodamine-labeled anti-phosphorylated amino acid antibody was obtained by introducing rhodamine to an anti-phosphorylated amino acid antibody (manufactured by Cosmo Bio Co. , Ltd.) in 0.1 M NaHCO₃ (pH 9) using rhodamine NHS (manufactured by Molecular Probes, Inc.). Subsequently, the above-mentioned support was washed with sterilized water, and fluorescence was measured by ScanArray 4000 (manufactured by GSI Lumonics Inc.).

Signals were clearly and specifically detected only in the spots containing phosphorylated peptide, so it was shown that the peptide was surely immobilized. Meanwhile, in the case of the controls (spots containing no peptide and spots containing an unphosphorylated peptide), no fluorescence was detected.

### Example 2

### <Immobilization of protein on support, evaluation of protein-immobilized support by antibody reaction>

Protein G (manufactured by Funakoshi Co., Ltd.) and BSA (manufactured by Sigma-Aldrich Corp.), 500 µg each, were respectively dissolved in a mixed solution (2 ml) of phosphate buffer (pH 7. 5) and DMF (N,N-dimethylformamide) (1:1 vol/vol), and an equal molar concentration of an oligonucleotide shown in SEQ ID NO: 4 obtained by introducing an amino group to the 5' -end of a polythymine derivative (manufactured by Glen Research Corporation, 12 bases) and 10-fold molar of DSS (manufactured by Pierce Biotechnology Inc.) were added, followed by incubation at 4°C for 10 hours. Subsequently, purification was performed using NAP5 column (manufactured by Amersham Biosciences), to thereby prepare a protein solution (50 µg/ml). Each of the above-mentioned protein solutions was spotted on predetermined positions on a support obtained by depositing gold on a glass plate. The amount of each spotted solution was 0.5 µl, and the spot size was 1 mm in diameter. Subsequently, the support was irradiated with an ultraviolet ray with a center wavelength of 335 nm using CRM-FA Spectro Iradiator (manufactured by JASCO Corporation). In this case, the irradiation intensity was 1.5 mW/cm², and the irradiation time was 120 seconds. Thereafter, the above-mentioned support was sha ken in water for 30 minutes and washed, followed by drying. On the other hand, as a control, anther support irradiated with no ultraviolet ray was also prepared.

On protein-immobilized parts on the above-mentioned protein-immobilized support, a solution containing Cy3-labeled anti-Protein G IgG (manufactured by Amersham Biosciences) (1 µg/200 µl IgG, 1 × PBS, 0.2% Triton X-100) was placed, and incubation was performed for 5 hours. Subsequently, the above-mentioned protein-immobilized support was washed with a solution of 1 × PBS-0. 2% Triton X-100, and fluorescence was measured by ScanArray 4000 (manufactured by GSI Lumonics Inc.).

Signals were clearly and specifically detected only in the spots containing Protein G on the support irradiated with an ultraviolet ray, so it was shown that the peptide was surely immobilized. Meanwhile, in the case of the controls (spots irradiated with no ultraviolet ray and spots containing BSA), no fluorescence was detected.

### Example 3

### <Immobilization of peptide on support using photocrosslinker, and evaluation of peptide-immobilized support by antibody reaction>

To 100 µl of the peptide solutions (1 pmol/µl) prepared in Example 1 was added 0.1 ml of a solution of psoralen (manufactured by Molecular Probes, Inc.) (200 µg/ml), and the solutions were mixed well, to thereby prepare a peptide solution containing psoralen (peptide 0.5 pmol/µl). Subsequently, each of the above-mentioned peptide solutions was spotted on predetermined positions on a glass support treated with a polymer compound containing a carbodiimide group. The amount of each spotted solution was 0.5 µl, and the spot size was 1 mm in diameter. Subsequently, irradiation was performed using HPW 125 Philips Lamp (manufactured by Philips Lightning) (center wavelength 365 nm) at an energy of 2.9 × 10¹⁶ quanta/sec for 60 minutes, and then the substrate was irradiated with 300 mJ/cm² ultraviolet ray using Uvstratalinker 2400 (manufactured by Stratagene, center wavelength 254 nm) from a distance of 16 cm. The irradiation time was 120 seconds. Thereafter, the above-mentioned support was shaken in water for 30 minutes and washed, followed by drying.

On the other hand, a solution containing no peptide (aqueous solution of disodium citrate) was also spotted on a support as a control in the same way as above, and the operations for immobilization as described above were performed.

On sample-immobilized parts on the above-mentioned support, a solution containing rhodamine-labeled anti-phosphorylated amino acid antibody (1 µg/100 µl antibody, 1 × PBS, 0.2% Tween 20, 1% BSA) was placed, and incubation was performed for 5 hours. The used rhodamine-labeled anti-phosphorylated amino acid antibody was obtained by introducing rhodamine to an anti-phosphorylated amino acid antibody (manufactured by Cosmo Bio Co., Ltd.) in 0.1 M NaHCO₃ (pH 9) using rhodamine NHS (manufactured by Molecular Probes, Inc.). Subsequently, the above-mentioned support was washed with sterilized water, and fluorescence was measured by ScanArray 4000 (manufactured by GSI Lumonics Inc.).

Signals were clearly and specifically detected only in the spots containing phosphorylated peptide, so it was shown that the peptide was surely immobilized. Meanwhile, in the case of the controls (spots containing no peptide and spots containing an unphosphorylated peptide), no fluorescence was detected.

### Example 4

### <Immobilization of peptide on support, and evaluation of peptide-immobilized support by antibody reaction>

A peptide (8 residues) having the amino acid sequence shown in SEQ ID NO: 5 and a peptide (8 residues) shown in SEQ ID NO: 6 in which serine is phosphorylated were synthesized using a peptide synthesizer. N-terminals of those peptides were acetylated on a solid-phase support (2-chlorotritylchloride resin) in accordance with a conventional method. The peptides having side chains with protecting groups [BOC (t-butoxycarbonyl) group and t-Bu (t-butyl) group] were cut off from the solid-phase support using a solution of acetic acid: trifluoroethanol: dichloromethane (1:2:7 vol/vol). To C-terminals of the peptides were introduced an oligonucleotide shown in SEQ ID NO: 4 obtained by introducing an amino group to the 5'-end of a polythymine derivative using 1,3-diisopropylcarbodiimide, 1-hydroxybenzotriazole, N,N-diisopropylethylamine, and 4-dimethylaminopyridine in DMF in accordance with a conventional method. Subsequently, the protecting groups of the peptides were subjected to deprotection with a solution of trifluoroacetic acid: water: diethanedithiol: thioanisole: phenol (10 ml:0.5 ml:0.25 ml:0.5 ml:0.75 g), and purification/concentration was performed using NAP5 column (manufactured by Amersham Biosciences), and each of the resultants was dissolved in an aqueous solution of 50 mM phosphate (pH 8.5), to thereby prepare a peptide solution (1 pmol/µl).

Each of the above-mentioned peptide solutions was spotted on predetermined positions on a glass support treated with a polymer compound containing a carbodiimide group. The amount of each spotted solution was 0.5 µl, and the spot size was 1 mm in diameter. Subsequently, the support was irradiated with an ultraviolet ray with a center wavelength of 335 nm using CRM-FA Spectro Iradiator (manufactured by JASCO Corporation). The irradiation intensity was 1.5mW/cm², and the irradiation time was 120 seconds. The irradiation time was 60 seconds. Thereafter, the above-mentioned support was shaken in water for 30 minutes and washed, followed by drying. On the other hand, as a control, another support irradiated with no ultraviolet ray was also prepared.

On peptide-immobilized parts on the above-mentioned peptide-immobilized support, a solution containing rhodamine-labeled anti-phosphorylated amino acid antibody (1µg/100 µl antibody, 1 × PBS, 0.2% Tween 20, 1% BSA) was placed, and incubation was performed for 5 hours. The used rhodamine-labeled anti-phosphorylated amino acid antibody was obtained by introducing rhodamine to an anti-phosphorylated amino acid antibody (manufactured by Cosmo Bio Co., Ltd.) in 0.1 M NaHC03 (pH 9) using rhodamine NHS (manufactured by Molecular Probes, Inc.). Thereafter, the above-mentioned peptide-immobilized support was washed with sterilized water, and fluorescence was measured by ScanArray 4000 (manufactured by GSI Lumonics Inc.).

Signals were clearly and specifically detected only in the spots containing phosphorylated peptide on the support irradiated with an ultraviolet ray, so it was shown that the peptide was surely immobilized. Meanwhile, in the case of the controls (spots irradiated with no ultraviolet ray and spots containing an unphosphorylated peptide), no fluorescence was detected.

### Example 5

### <Immobilization of peptide on support, and evaluation of peptide-immobilized support by antibody reaction>

The peptide solutions prepared in Example 4 (1 pmol/µl) were spotted on predetermined positions on a glass support (manufactured by Telechem International, Inc.) treated with aminosilane. The amount of each spotted solution was 0.5 µl, and the spot size was 1 mm in diameter. Subsequently, the support was irradiated with an ultraviolet ray with a center wavelength of 335 nm using CRM-FA Spectro Iradiator (manufactured by JASCO Corporation). The irradiation intensity was 1.5 mW/cm², and the irradiation time was 120 seconds. Thereafter, the above-mentioned support was shaken in water for 30 minutes and washed, followed by drying. On the other hand, as a control, another support irradiated with no ultraviolet ray was also prepared.

On peptide-immobilized parts on the above-mentioned peptide-immobilized support, a solution containing rhodamine-labeled anti-phosphorylated amino acid antibody (1 µg/100 µl antibody, 1 PBS, 0.2% Tween 20, 1% BSA) was placed, and incubation was performed for 5 hours. The used rhodamine-labeled anti-phosphorylated amino acid antibody was obtained by introducing rhodamine to an anti-phosphorylated amino acid antibody (manufactured by Cosmo Bio Co., Ltd.) in 0.1 M NaHCO₃ (pH 9) using rhodamine NHS (manufactured by Molecular Probes, Inc.). Thereafter, the above-mentioned peptide-immobilized support was washed with sterilized water, and fluorescence was measured by ScanArray 4000 (manufactured by GSI Lumonics Inc.).

Signals were clearly and specifically detected only in the spots containing phosphorylated peptide on the support irradiated with an ultraviolet ray, so it was shown that the peptide was surely immobilized. Meanwhile, in the case of the controls (spots irradiated with no ultraviolet ray and spots containing an unphosphorylated peptide), no fluorescence was detected.

### Example 6

### <Immobilization of peptide on support with photoreactive group, and evaluation of peptide-immobilized support by antibody reaction>

In accordance with a conventional method, 4-benzoylbenzoic chloride was synthesized from 4-benzoylbenzoic acid. 4-Benzoylbenzoic chloride (30 g) was dissolved in chloroform (500 ml), and an aqueous solution of 6-aminohexanoic acid (17 g/400 ml 1N NaOH) was added by dropwise at 0°C, followed by stirring at room temperature for 60 minutes. 12N HCl (40 ml) was added to the reaction mixture, and the organic solvent phase was extracted. The organic solvent phase was dried by vacuum concentration. Subsequently, 1,4-dioxane (500 ml) was added to the resultant dried product to dissolve it, and N-hydroxysuccinimide (10.7 g) and 1,3-dicyclohexylcarbodiimide (20.1 g) were added, followed by stirring at room temperature for 18 hours. The mixture was filtered, and then the solution was concentrated under reduced pressure, to thereby yield 25 g of N-succinimidyl 6-(4-benzoylbenzamide)hexanoate.

In accordance with a conventional method, a peptide (8 residues) having the amino acid sequence shown in SEQ ID NO: 5 was synthesized using a peptide synthesizer. In addition, a peptide (8 residues) shown in SEQ ID NO: 6 in which serine is phosphorylated was also synthesized. Subsequently, 1 M sodium bicarbonate buffer (pH 9.0) (0.5 ml) was added to the peptides (10 nmol) respectively to dissolve them, and N-succinimidyl 6-(4-benzoylbenzamide)hexanoate (100 nmol) dissolved in DMF was added, followed by stirring at room temperature for 18 hours. Thereafter, purification/concentration was performed using NAP5 column (manufactured by Amersham Biosciences), and the resultant was dissolved in an aqueous solution of 50 mM phosphate (pH 8.5), to thereby prepare a peptide solution (1 pmol/µl).

Each of the above-mentioned peptide solutions was spotted on predetermined positions on a commercially available polypropylene glass support (polypropylene plate manufactured by Takiron Co., Ltd.) The amount of each spotted solution was 0.5 µl, and the spot size was 1 mm in diameter. Subsequently, the support was irradiated with an ultraviolet ray with a center wavelength of 335 nm using CRM-FA Spectro Iradiator (manufactured by JASCO Corporation). The irradiation intensity was 1.5 mW/cm², and the irradiation time was 120 seconds. Thereafter, the above-mentioned support was shaken in water for 30 minutes and washed, followed by drying. On the other hand, as a control, another support irradiated with no ultraviolet ray was also prepared.

On peptide-immobilized parts on the above-mentioned peptide-immobilized support, a solution containing rhodamine-labeled anti-phosphorylated amino acid antibody (1 µg/100 µl antibody, 1 × PBS, 0.2% Tween 20, 1% BSA) was placed, and incubation was performed for 5 hours. The used rhodamine-labeled anti-phosphorylated amino acid antibody was obtained by introducing rhodamine to an anti-phosphorylated amino acid antibody (manufactured by Cosmo Bio Co., Ltd.) in 0.1 M NaHCO₃ (pH 9) using rhodamine NHS (manufactured by Molecular Probes, Inc.). Thereafter, the above-mentioned peptide-immobilized support was washed with sterilized water, and fluorescence was measured by ScanArray 4000 (manufactured by GSI Lumonics Inc.).

Signals were clearly and specifically detected only in the spots containing phosphorylated peptide on the support irradiated with an ultraviolet ray, so it was shown that the peptide was surely immobilized. Meanwhile, in the case of the controls (spots irradiated with no ultraviolet ray and spots containing an unphosphorylated peptide), no fluorescence was detected.

### Example 7

### <Immobilization of protein on support with photoreactive group, and evaluation of protein-immobilized support by antibody reaction>

1 M sodium bicarbonate buffer (pH 9.0) (1 ml) was added to Protein G (manufactured by Funakoshi Co., Ltd.) and BSA (manufactured by Sigma-Aldrich Corp.) respectively, 500 µg each, and a solution of N-succinimidyl 6-(4-benzoylbenzamide)hexanoate (100 nmol) in DMF prepared in Example 6 was added, followed by stirring at room temperature for 18 hours. Subsequently, purification/concentration was performed using NAP5 column (manufactured by Amersham Biosciences), and the resultant was dissolved in an aqueous solution of 50 mM phosphate (pH 8.5), to thereby prepare a protein solution (30 µg/ml).

Each of the resultant proteins was spotted on predetermined positions on a commercially available polycarbonate support (general polycarbonate plate manufactured by Takiron Co., Ltd.). The amount of each spotted solution was 0.5 µl, and the spot size was 1 mm in diameter. Subsequently, the support was irradiated with an ultraviolet ray with a center wavelength of 335 nm using CRM-FA Spectro Iradiator (manufactured by JASCO Corporation). The irradiation intensity was 1.5 mW/cm², and the irradiation time was 120 seconds. Thereafter, the above-mentioned support was shaken in water for 30 minutes and washed, followed by drying. Meanwhile, as a control, another support irradiated with no ultraviolet ray was also prepared.

On protein-immobilized parts on the above-mentioned protein-immobilized support, a solution containing Cy3-labeled anti-Protein G IgG (manufactured by Amersham Biosciences) (0.5 µg/200 µl IgG, 1 × PBS, 0.2% Triton X-100) was placed, and incubation was performed for 5 hours. Subsequently, the above-mentioned protein-immobilized support was washed with a solution of 1 × PBS-0.2% Triton X-100, and fluorescence was measured by ScanArray 4000 (manufactured by GSI Lumonics Inc.).

Signals were clearly and specifically detected only in the spots containing Protein G on the support irradiated with an ultraviolet ray, so it was shown that the protein was surely immobilized. Meanwhile, in the case of the controls (spots irradiated with no ultraviolet ray and spots containing BSA), no fluorescence was detected.

### Example 8

### [Immobilization of sugar on support, and evaluation of sugar-immobilized support by utilizing interaction between lectin and sugar>

2-Aminoethyl-β-D-galactopyranoside (manufactured by Mitani Sangyo Co., Ltd.) and an oligonucleotide (10 bases) shown in SEQ ID NO: 7 obtained by introducing an amino group to the 5'-end of a polymer including a deoxythymidylic acid and a deoxycytidylic acid were dissolved in methanol: isopropylalcohol: sterilized water: DMSO (5:5:5:1), and a solution of tributylamine (manufactured by Wako Pure Chemical Industries, Ltd.) was added to adjust the pH to 8.0. Subsequently, 2-fold molar of DSS (manufactured by Pierce Biotechnology Inc.) was added, and incubation was performed at 42°C for 5 hours. Thereafter, purification was performed using reversed-phase HPLC (manufactured by Waters Corporation, µBondaspphere, C8 300A, 3.9 × 150), followed by concentration, and the resultant was dissolved in an aqueous solution of 45mM diammonium citrate, to thereby prepare a sugar solution (1 pmol/µl).

The above-mentioned sugar solution and buffer solution were separately spotted on predetermined positions on a flat-bottom 96-well polystyrene microtiter plate (manufactured by Tech-Jam). The amount of each spotted solution was 0.5 µl, and the spot size was 1 mm in diameter. Subsequently, the support was irradiated with 80 mJ/cm² ultraviolet ray using Uvstratalinker 2400 (manufactured by Stratagene, center wavelength 254 nm) from a distance of 16 cm. The irradiation time was 30 seconds. Thereafter, the above-mentioned support was shaken in water for 30 minutes and washed, followed by drying. On the other hand, as a control, another support irradiated with no ultraviolet ray was also prepared.

FITC-labeled lectin (derived from Sophora japonica), which had been prepared according to the method of A. McPherson et al. (McPherson, A.; Hankins, C.N.; Shannon, L.J. Biol. Chem. 1987, 262, 1791-1794), was dissolved in 1 × PBST solution containing 1% BSA at a concentration of 1 mM. Subsequently, the solution containing FITC-labeled lectin was placed on sugar-immobilized parts on the above-mentioned support, and incubation was performed at room temperature for 12 hours. Thereafter, the above-mentioned sugar-immobilized support was washed with sterilized water, and fluorescence was measured using FLA 5000 (manufactured by Fuji Photo Film Co., Ltd.).

Signals were clearly and specifically detected only in the spots containing galactose on the support irradiated with an ultraviolet ray, so it was shown that the sugar was surely immobilized. Meanwhile, in the case of the controls (spots irradiated with no ultraviolet ray), only fluorescence intensity having the same intensity as that of background noise (buffer solution) was detected.

### Example 9

### <Immobilization ofpeptide on polyacryl amide support, and evaluation of peptide-immobilized support by enzyme reaction>

A peptide (6 residues) having the amino acid sequence shown in SEQ ID NO: 8 was synthesized using a peptide synthesizer. The above-mentioned synthesized peptide and an oligonucleotide obtained by introducing an amino group to the 5'-end of the oligonucleotide (10 bases) shown in SEQ ID NO: 7 were dissolved in 0.1 M sodium bicarbonate buffer (pH 8.0) at equal molar concentrations, and 10-fold molar of DSS (manufactured by Pierce Biotechnology Inc.) dissolved in DMF was added, followed by incubation at 37°C for 2 hours. Subsequently, purification was performed using reversed-phase HPLC (manufactured by Waters Corporation, µBondaspphere, C8 300A, 3.9 × 150), followed by concentration, and the resultant was dissolved in an aqueous solution of 45 mM diammonium citrate, to thereby prepare a peptide solution (5 pmol/µl).

Each of the above-mentioned peptide solutions was spotted on 3D-Link™ Activated Slide (manufactured by Surmodics, Inc.), which is a slide having a surface with polyacrylamide, using Nano-Plotter™ (manufactured by GeSim). The spot size was 0.3 mm in diameter. Subsequently, the support was irradiated with 60 mJ/cm² ultraviolet ray using Uvstratalinker 2400 (manufactured by Stratagene, center wavelength 254 nm) from a distance of 16 cm. The irradiation time was 24 seconds. Thereafter, the above-mentioned support was shaken in water for 30 minutes and washed, followed by drying. On the other hand, as a control, a solution containing no peptide (aqueous solution of disodium citrate) was also spotted on the support in the same way, and the operations for immobilization as described above were performed.

On sample-immobilized parts on the above-mentioned peptide-immobilized support, buffer containing tyrosine p60^{c-src} kinase (manufactured by Upstate) [2 U/50 µl enzyme, 25 mM Tris (pH 7.4), 15 mM MgCl₂, 7 mM MnCl₂, 0.5 mM EGTA, 100 µM ATP] was placed, and incubation was performed for 5 hours. Subsequently, the above-mentioned sample-immobilized support was washed with a solution of 1 × PBS-0.2% Tween 20, and buffer (1 µg/100 µl antibody, 1 × PBS, 0.2% Tween 20, 1% BSA) containing FITC-labeled anti-phosphotyrosine (manufactured by Sigma-Aldrich Corp.) was placed on peptide-immobilized parts on the above-mentioned peptide-immobilized support, and incubation was performed for 2 hours. Thereafter, the above-mentioned peptide-immobilized support was washed with a solution of 1 × PBS-0.2% Tween 20, and fluorescence was measured using FLA 5000 (manufactured by Fuji Photo Film Co., Ltd.).

On the other hand, buffer [25 mM Tris (pH 7.4), 15 mM MgCl₂, 7 mM MnCl₂, 0.5 mM EGTA, 100 µM ATP] not containing tyrosine p60^{c-src}kinase was placed, followed by incubation for 5 hours, and the above-mentioned FITC-labeled anti-phosphotyrosine was placed, followed by incubation for 2 hours, to thereby prepare another peptide-immobilized support.

Signals were clearly and specifically detected only in the spots containing the peptide on the support incubated with buffer containing tyrosine p60^{c-src}kinase, so it was shown that the peptide was surely immobilized, and the enzyme reaction could be specifically detected. Meanwhile, in the case of the controls (spots containing the peptide on the support incubated with buffer not containing tyrosine p60^{c-src}kinase and spots containing no peptide), no fluorescence was detected.

### Example 10

### <Immobilization of peptide on nitrocellulose support, and evaluation of peptide-immobilized support by enzyme reaction>

The peptide solution prepared in Example 9 (5 pmol/µl) was spotted on FAST Slide (manufactured by Schleicher & Schuell BioScience), which is a slide having a surface with nitrocellulose, using Nano-Plotter™ (manufactured by GeSim). The spot size was 0.4 mm in diameter. Subsequently, the support was irradiated with 120 mJ/cm² ultraviolet ray using Uvstratalinker 2400 (manufactured by Stratagene, center wavelength 254 nm) from a distance of 16 cm. The irradiation time was 48 seconds. Thereafter, the above-mentioned support was shaken in a solution of 3% BSA for 30 minutes and washed, followed by drying. On the other hand, as a control, a solution containing no peptide (aqueous solution of disodium citrate) was also spotted on the support in the same way, and the operations for immobilization as described above were performed.

On sample-immobilized parts on the above-mentioned peptide-immobilized support, buffer containing tyrosine p60^{c-src} kinase (manufactured by Upstate) [2 U/50 µl enzyme, 25 mM Tris (pH 7.4), 15 mM MgCl₂, 7 mM MnCl₂, 0.5 mM EGTA, 100 µM ATP] was placed, and incubation was performed for 5 hours. Subsequently, the above-mentioned peptide-immobilized support was washed with a solution of 1 × PBS-0.2% Tween 20, and buffer containing FITC-labeled anti-phosphotyrosine (manufactured by Sigma-Aldrich Corp.) (1 µg/100 µl antibody, 1 × PBS, 0.2% Tween 20, 1% BSA) was placed on peptide-immobilized parts on the above-mentioned sample-immobilized support, and incubation was performed for 2 hours. Thereafter, the above-mentioned peptide-immobilized support was washed with a solution of 1 × PBS-0.2% Tween 20, and fluorescence was measured using FLA 5000 (manufactured by Fuji Photo Film Co., Ltd.).

On the other hand, buffer [25 mM Tris (pH 7.4), 15 mM. MgCl₂, 7 mM MnCl₂, 0.5 mM EGTA, 100 µM ATP] not containing tyrosine p60^{c-src}kinase was placed, followed by incubation for 5 hours, and the above-mentioned FITC-labeled anti-phosphotyrosine was placed, followed by incubation for 2 hours, to thereby prepare another peptide-immobilized support.

Signals were clearly and specifically detected only in the spots containing the peptide on the support incubated with buffer containing tyrosine p60^{c-src}kinase, so it was shown that the peptide was surely immobilized, and the enzyme reaction could be specifically detected. Meanwhile, in the case of the controls (spots containing the peptide on the support incubated with buffer not containing tyrosine p60^{c-src}kinase and spots containing no peptide), no fluorescence was detected.

### Example 11

### <Immobilization of peptide on acrylamide gel support, and evaluation of peptide-immobilized support by enzyme reaction>

A slide, which is a slide having a surface with acrylamide gel, was prepared according to the method of Nallur et al. [Nallur G, Luo C, Fang L, Cooley S, Dave V, Lambert J, Kukanskis K, Kingsmore S, Lasken R, Schweitzer B. (2001) Signal amplification by rolling circle amplification on DNA microarrays. Nucleic Acids Res., 29, e118.]. The peptide solution prepared in Example 9 (5 pmol/µl) was spotted on the above-mentioned slide, which is a slide having a surface with acrylamide gel, using Nano-Plotter™ (manufactured by GeSim). The spot size was 0.5 mm in diameter. Subsequently, the support was irradiated with 120 mJ/cm² ultraviolet ray using Uvstratalinker 2400 (manufactured by Stratagene, center wavelength 254 nm) from a distance of 16 cm. The irradiation time was 48 seconds. Thereafter, the above-mentioned support was shaken in a solution of 3% BSA for 30 minutes and washed, followed by drying. On the other hand, as a control, a solution containing no peptide (aqueous solution of disodium citrate) was also spotted on the support in the same way, and the operations for immobilization as described above were performed.

On sample-immobilized parts on the above-mentioned peptide-immobilized support, buffer containing tyrosine p60^{c-src}kinase (manufactured by Upstate) [2 U/50 µl enzyme, 25 mM Tris (pH 7.4), 15 mM MgCl₂, 7 mM MnCl₂, 0.5 mM EGTA, 100 µM ATP] was placed, and incubation was performed for 5 hours. Subsequently, the above-mentioned peptide-immobilized support was washed with a solution of 1 × PBS-0.2% Tween 20, and buf fer containing FITC-labeled anti-phosphotyrosine (manufactured by Sigma-Aldrich Corp.) (1 µg/100 µl antibody, 1 × PBS, 0.2% Tween 20, 1% BSA) was placed on sample-immobilized parts on the above-mentioned peptide-immobilized support, and incubation was performed for 2 hours. Thereafter, the above-mentioned peptide-immobilized support was washed with a solution of 1 × PBS-0.2% Tween 20, and fluorescence was measured using FLA 5000 (manufactured by Fuji Photo Film Co., Ltd.).

On the other hand, buffer [25 mM Tris (pH 7.4), 15 mM MgCl₂, 7 mM MnCl₂, 0.5 mM EGTA, 100 µM ATP] not containing tyrosine p60^{c-src}kinase was placed, followed by incubation for 5 hours, and the above-mentioned FITC-labeled anti-phosphotyrosine was placed, followed by incubation for 2 hours, to thereby prepare another peptide-immobilized support.

Signals were clearly and specifically detected only in the spots containing the peptide on the support incubated with buffer containing tyrosine p60^{c-src}kinase, so it was shown that the peptide was surely immobilized, and the enzyme reaction could be specifically detected. Meanwhile, in the case of the controls (spots containing the peptide on the support incubated with buffer not containing tyrosine p60^{c-src} kinase and spots containing no peptide), no fluorescence was detected.

### Example 12

### <Immobilization of peptide on polylysine support, evaluation of peptide-immobilized support by enzyme reaction>

A peptide (6 residues) having the amino acid sequence shown in SEQ ID NO: 8 was synthesized using a peptide synthesizer. The above-mentioned synthesized peptide and an oligonucleotide obtained by introducing an amino group to the 5' -end of the oligonucleotide (20 bases) shown in SEQ ID NO: 9 were dissolved in 0.1 M sodium bicarbonate buffer (pH 8.0) at equal molar concentrations, and 10-fold molar of DSS (manufactured by Pierce Biotechnology Inc.) dissolved in DMF was added, followed by incubation at 37°C for 2 hours. Subsequently, purification was performed using reversed-phase HPLC (manufactured by Waters Corporation, µBondaspphere, C8 300A, 3.9 × 150), followed by concentration, and the resultant was dissolved in an aqueous solution of 45 mM diammonium citrate, to thereby prepare a peptide solution (10 pmol/µl).

The above-mentioned peptide solution was spotted on a slide, which is a slide having a surface with polylysine, using SP-BIO (manufactured by Hitachi, Ltd.). The spot size was 0.3 mm in diameter. Subsequently, the support was irradiated with 240 mJ/cm² ultraviolet ray using Uvstratalinker 2400 (manufactured by Stratagene, center wavelength 254 nm) from a distance of 16 cm. The irradiation time was 96 seconds. Thereafter, the above-mentioned support was shaken in a solution of 1% BSA for 30 minutes and washed, followed by drying. On the other hand, as a control, a solution containing no peptide (aqueous solution of disodiumcitrate) was also spotted on the support in the same way, and the operations for immobilization as described above were performed.

On sample-immobilized parts on the above-mentioned peptide-immobilized support, buffer containing tyrosine p60^{c-src}kinase (manufactured by Upstate) [2 U/50 µl enzyme, 25 mM Tris (pH 7.4), 15 mM MgCl₂, 7 mM MnCl₂, 0.5 mM EGTA, 100 µM ATP] was placed, and incubation was performed for 5 hours. Subsequently, the above-mentioned peptide-immobilized support was washed with a solution of 1 × PBS-0.2% Tween 20, and buffer containing FITC-labeled anti-phosphotyrosine (manufactured by Sigma-Aldrich Corp.) (1 µg/100 µl antibody, 1 × PBS, 0.2% Tween 20, 1% BSA) was placed on sample-immobilized parts on the above-mentioned peptide-immobilized support, and incubation was performed for 2 hours. Thereafter, the above-mentioned peptide-immobilized support was washed with a solution of 1 × PBS-0.2% Tween 20, and fluorescence was measured using FLA 5000 (manufactured by Fuji Photo Film Co., Ltd.).

On the other hand, buffer [25 mM Tris (pH 7.4), 15 mM MgCl₂, 7 mM MnCl₂, 0.5 mM EGTA, 100 µM ATP] not containing tyrosine p60^{c-src} kinase was placed, followed by incubation for 5 hours, and the above-mentioned FITC-labeled anti-phosphotyrosine was placed, followed by incubation for 2 hours, to thereby prepare another peptide-immobilized support.

Signals were clearly and specifically detected only in the spots containing the peptide on the support incubated with buffer containing tyrosine p60^{c-src}kinase, so it was shown that the peptide was surely immobilized, and the enzyme reaction could be specifically detected. Meanwhile, in the case of the controls (spots containing the peptide on the support incubated with buffer not containing tyrosine p60^{c-src} kinase and spots containing no peptide), no fluorescence was detected.

### Example 13

### <Immobilization of peptide on polycarbodiimide support, elongation reaction by SPOT method, and evaluation of peptide-immobilized support by antibody reaction>

According to the method described in Example 1, the peptide (Ala-Ala: 2 residues) bound to polynucleotide shown in SEQ ID NO: 10 was prepared, and the above-mentioned polynucleotide-bound peptide was immobilized on a glass substrate treated with a polymer compound having a carbodiimide group.

According to the method of Tegge et al. (Tegge W, Frank R: Determination of Cyclic Nucleotide-Dependent Protein Kinase Substrate Specificity by the Use of Peptide Libraries on Cellulose Paper. Biochemistry 1995, 34: 10569-105777), amino acid-coupling (elongation) reaction was repeated at sample-immobilized parts on the above-mentioned glass substrate to synthesize the peptide (12 bases) shown in SEQ ID NO: 11 on the above-mentioned glass substrate. In this case, the elongation reaction was performed using an amino acid having N-terminal protected with an Fmoc group and side chain protected with a t-Bu, trityl, or Boc group and using, as coupling agents, diisopropyl carbodiimide (manufactured by Wako Pure Chemical Industries, Ltd.) and hydroxybenzotriazole (manufactured by Wako Pure Chemical Industries, Ltd.) at equal molar concentrations. Meanwhile, in each elongation reaction, it was confirmed that the sample-immobili zed parts turned blue by treating sample-immobilized parts on the above-mentioned glass substrate with Bromophenol blue (0.1 mg/ml) dissolved in a DMF solution, and then the next elongation reaction was performed.

Subsequently, the N-terminal of the synthesized peptide was acetylated by treating the sample-immobilized parts on the above-mentioned glass substrate with a solution of 2% acetic anhydride/DMF, and the sample-immobilized portions on the above-mentioned glass substrate were further treated with a solution of dichloromethane/trifluoroacetic acid (1:1) containing 3% triisobutylsilane and 2% water at room temperature for 2 hours to perform deprotection reaction for a side chain of the peptide.

Meanwhile, as a control, there was also prepared a sample that was not subjected to the above-mentioned elongation reaction after immobilization of the above-mentioned peptide bound with a polynucleotide on the above-mentioned glass substrate.

On sample-immobilized parts on the above-mentioned support, a solution containing Cy3-labeled anti-angiotensin I antibody (2.2 µg/100 µl antibody, 1 × PBS, 0.2% Tween 20, 1% BSA) was placed, and incubation was performed for 2 hours. The used Cy3-labeled anti-angiotensin I antibody was prepared by introducing Cy3 to anti-angiotensin I antibody (purchased from Cosmo Bio Co., Ltd.) in 0. 1 M NaHCO₃ (pH 9) using Cy3-NHS (manufactured by GE Healthcare) . Subsequently, the above-mentioned support was washed with sterilized water, and fluorescence was measured by ScanArray 4000 (manufactured by GSI Lumonics Inc.).

Signals were clearly and specifically detected only in the spots where the elongation reactions were performed, so it was shown that the peptide was surely immobilized. Meanwhile, in the case of the control (spots where the elongation reactions were not performed), no fluorescence was detected.

### Industrial Applicability

The protein-immobilized substrate of the present invention may be a protein-immobilized substrate effective for application such as a protein tip having excellent reproducibility and quantitative ability because the protein can be bound to a substrate or a carrier provided on the substrate.

## Claims

1. A biomolecule (excluding a nucleic acid) to be immobilized and used for a method of detecting a substance capable of interacting with the biomolecule using the immobilized biomolecule, wherein the biomolecule is bound to a compound having a group capable of binding onto a substrate for immobilizing a biomolecule to which the biomolecule is immobilized or a carrier provided on the substrate.

2. A biomolecule according to Claim 1, wherein the compound having a group capable of binding onto a substrate for immobilizing a biomolecule or a carrier provided on the substrate is a polymer that includes a compound having an unsaturated bond.

3. A biomolecule according to Claim 2, wherein the polymer has an average degree of polymerization of 2 or more to 1,000,000 or less.

4. A biomolecule according to Claim 2 or 3, wherein a monomer constituting the polymer is a nucleotide.

5. A biomolecule according to Claim 1, wherein the compound having a group capable of binding onto a substrate for immobilizing a biomolecule or a carrier provided on the substrate is a compound having at least one photoreactive group, and selected from compounds each having a nitrene precursor, carbene precursor, or ketone group.

6. A biomolecule according to any one of Claims 1 to 5, wherein the biomolecule is selected from a protein, sugar, antigen, antibody, peptide, and enzyme.

7. A substrate for immobilizing a biomolecule, comprising:
a substrate for immobilizing a biomolecule; and a biomolecule according to any one of Claims 1 to 6 immobilized on the substrate.

8. A method of producing a substrate for immobilizing a biomolecule, comprising: contacting the biomolecule according to any one of Claims 1 to 6 with a substrate for immobilizing a biomolecule; and irradiating a contact portion with an electromagnetic ray.

9. A method of detecting a substance capable of interacting with an immobilized biomolecule using the immobilized biomolecule, wherein the substrate for immobilizing a biomolecule according to Claim 7 is used.


SEQUENCE LISTING

<110> Nisshinbo Industries, Inc.

<120> IMMOBILIZED BIOMOLECULE AND METHOD OF DETECTING SUBSTANCE CAPABLE OF INTERACTING WITH BIOMOLECULE

<130> OP-C4313-PCT

<150> JP 2004-61798
<151> 2004-3-5
<150> JP 2004-319087
<151> 2004-11-2

<160> 11

<210> 1
<211> 7
<212> PRT
<213> Artificial Sequence

<220>
<223> Synthetic Protein

<400> 1
Tyr Ile Tyr Gly Ser Phe Lys
1               5

<210> 2
<211> 7
<212> PRT
<213> Artificial Sequence

<220>
<221> Phosphorylation
<222> (1, 2, 3, 4, 5, 6, 7)
<223> Synthetic Protein

<400> 2
Tyr Tyr Tyr Tyr Tyr Tyr Tyr
1               5

<210> 3
<211> 10
<212> DNA
<213> Artificial Sequence

<220>
<223> Synthetic DNA

<400> 3
tttttttttt      10

<210> 4
<211> 12
<212> DNA
<213> Artificial Sequence

<220>
<221> 4-thiothymine
<222> (1,2,3,4,5,6,7,8,9,10,11,12)
<223> Synthetic DNA

<400> 4
tttttttttt tt      12

<210> 5
<211> 8
<212> PRT
<213> Artificial Sequence

<220>
<223> Synthetic Protein

<400> 5
Ala Leu Arg Arg Ala Ser Leu Gly
1               5

<210> 6
<211> 8
<212> PRT
<213> Artificial Sequence

<220>
<221> Phosphorylation
<222> (6)
<223> Synthetic Protein

<400> 6
Ala Leu Arg Arg Ala Ser Leu Gly
1               5

<210> 7
<211> 10
<212> DNA
<213> Artificial Sequence

<220>
<223> Synthetic DNA

<400> 7
cccccttttt      10

<210> 8
<211> 6
<212> PRT
<213> Artificial Sequence

<220>
<223> Synthetic Protein

<400> 8
Tyr Ile Tyr Gly Ser Phe
1               5

<210> 9
<211> 20
<212> DNA
<213> Artificial Sequence

<220>
<223> Synthetic DNA

<400> 9
tatatatata tatatatata      20

<210> 10
<211> 14
<212> DNA
<213> Artificial Sequence

<220>
<223> Synthetic DNA

<400> 10
CCCCCCCTTT TTTT      14

<210> 11
<211> 12
<212> PRT
<213> Artificial Sequence

<220>
<223> Synthetic Protein

<400> 11
Asp Arg Val Tyr Ile His Pro Phe His Leu Ala Ala
1               5                   10